# EUROPEAN PATENT SPECIFICATION

(11) **EP 3 272 241 B1**
(45) Date of publication and mention of the grant of the patent: **23.09.2020**
(21) Application number: 16765120.7
(22) Date of filing: 18.03.2016
(51) Int. Cl.: A42B 3/24, A61F 9/02, B32B 27/18, B32B 27/36, G02C 11/00

(54) **ATTACHMENT SHEET, HELMET, GOGGLES, AND METHOD FOR ATTACHING ANTI-FOG SHEET**
BEFESTIGUNGSFOLIE, HELM, BRILLE UND VERFAHREN ZUR BEFESTIGUNG EINER ANTIBESCHLAGSFOLIE
FEUILLE DE FIXATION, CASQUE, LUNETTES ET PROCÉDÉ POUR FIXER UNE FEUILLE ANTI-BUÉE

(30) Priority: 19.03.2015 JP 2015056792
(43) Date of publication of application: 24.01.2018
(73) Proprietor: Wins Japan Co. Ltd., Kanazawa-shi, Ishikawa 921-8163 (JP); Yamamoto Kogaku Co., Ltd., Osaka 577-0056 (JP)
(72) Inventor: KATAOKA Tadashi, Kanazawa-shi Ishikawa 921-8163 (JP); KOBAYASHI Hirokazu, Higashiosaka City Osaka 577-0056 (JP); YOKOCHI Junpei, Higashiosaka City Osaka 577-0056 (JP)
(74) Representative: Goddar, Heinz J.
(86) International application number: PCT/JP2016/058802
(87) International publication number: WO 2016/148291

(56) References cited:
- WO-A1-2009/056608
- JP-A- H 073 515
- JP-A- H05 222 227
- JP-A- H05 222 227
- JP-A- S60 250 927
- JP-A- 2007 210 138
- JP-A- 2010 518 270
- JP-A- 2011 189 171
- JP-A- 2011 252 258
- US-A- 5 765 235

## Description

### TECHNICAL FIELD

The present invention relates to a mounting sheet, a helmet, goggles, and a method for mounting an anti-fog sheet.

### BACKGROUND ART

WO 2008/096178 A1 (JP 2010/518270 A1) discloses a visor assembly including a sealing member which spaces an overlay-sheet from a visor and forms a sealed compartment between the visor and the overlay-sheet.

WO 2009/040581A1 (JP 2010/540787 A1) discloses a method for producing a seal or spacer extending along a portion of a periphery of a viewing area of a visor by injection molding of a quick-setting elastomer resin.

WO 2009/056608 A1 relates to a visor structure for a protective device such as a motorcycle helmet. The structure according to the invention comprises an outer visor made of transparent plastic material and provided with a front surface and a rear surface opposite to the front surface. An inner visor made of plastic material with anti-fogging properties is constrained to the outer one so as to face to said rear face. This latter comprises at least one recess which defines constraining portions each of which constrains a lateral edge of the inner visor. The inner visor having a different curvature with respect to that of said rear surface of the outer visor.

JP H05-222227 A relates to an anti-fogging film which can be fixed to the surfaces of clear formed articles without requiring a strong adhesive because it causes reduced warpage and deformation by moisture absorption and can readily be peeled off. On the one surface of polycarbonate film of 50 to 150mum thickness, an anti-fogging, hydrophilic film is formed by curing a hydrophilic anti-fogging agent curable with activated energy beams. The thickness of the anti- fogging layer is more than 10% and less than 40 % of the polycarbonate film stated above. Either one of the back surface of the anti-fogging-treated polycarbonate film or the surface of a clear formed article is wetted with a liquid and the not-anti-fogging surface is stuck to the surface of the clear formed article to keep out the surface from fogging.

### SUMMARY OF INVENTION

### TECHNICAL PROBLEMS

It is an object of the present invention to provide a mounting sheet which can be easily mounted to a visor shield in a helmet, a lens in goggles, or the like and is less likely to come off.

### SOLUTIONS TO PROBLEMS

A mounting sheet according to the present invention is a mounting sheet which is mounted to a concave curved surface in a state where the mounting sheet is deflected along the curved surface. The mounting sheet has a base material sheet, which is formed of polycarbonate having a thickness of 0.1 mm or more and has a curvature smaller than that of the concave curved surface when not mounted to the concave curved surface, and an anti-fog layer provided on at least one of both surfaces of the base material sheet, which does not face the concave curved surface when mounted to the concave curved surface. A total thickness of the base material sheet and the anti-fog layer is 5.0 mm or less. The concave curved surface is an inner wall surface of a visor shield or a goggle lens, and the thickness of the base material sheet is 0.2 mm or more and 1.0 mm or less.

Preferably, the concave curved surface is a smooth three-dimensional concave curved surface, and the thickness of the base material sheet is 0.2 mm or more and 0.6 mm or less.

Preferably, the anti-fog layer is a hydrophilic anti-fog film chemically bonded to a surface of the base material sheet.

A helmet according to the present invention has a visor shield, a mounting sheet mounted to a concave curved surface of the visor shield, and fixing means for fixing the mounting sheet of the invention to the concave curved surface of the visor shield. The fixing means is at least provided at both ends in a longitudinal direction of the mounting sheet and fixes the mounting sheet to the visor shield at the both ends.

Preferably, the fixing means is an elastic adhesive layer disposed between the visor shield and the mounting sheet and having a convex curved surface on a surface facing at least one of the visor shield and the mounting sheet.

Preferably, the visor shield is formed of polycarbonate, and the fixing means sandwiches and fixes both ends of the mounting sheet in a deflected state.

Preferably, the fixing means is at least an elastic adhesive layer continuously provided along an outer edge portion of the mounting sheet.

Preferably, the fixing means is an elastic adhesive layer which is highly raised or has a large width at both longitudinal end portions of the mounting sheet, rather than at a longitudinal center portion of the mounting sheet.

Goggles according to the present invention has a goggle lens, a mounting sheet mounted to a concave curved surface of the goggle lens, and fixing means for fixing the mounting sheet of the invention to the concave curved surface of the goggle lens. The fixing means is at least provided at both ends in a longitudinal direction of the mounting sheet and fixes the mounting sheet to the goggle lens at the both ends.

A method for mounting an anti-fog sheet according to the present invention includes the steps of forming an anti-fog layer on at least one surface of a polycarbonate base material sheet having a thickness of 0.1 mm or more and 1.0 mm or less and fixing the polycarbonate base material sheet to a concave curved inner wall surface of a visor shield or the goggle lens in a state where the polycarbonate base material sheet is deflected such that the surface formed with the anti-fog layer is a concave curved surface. A total thickness of the base material sheet (100) and the anti-fog layer (105) is 5.0 mm or less.

### ADVANTAGEOUS EFFECTS OF INVENTION

The present invention provides a mounting sheet which can be easily mounted to a visor shield in a helmet, a lens in goggles, or the like and is less likely to come off.

### BRIEF DESCRIPTION OF DRAWINGS

Fig. 1 is a diagram exemplifying an overall configuration of a helmet 1.
Fig. 2 is a diagram schematically representing a state where a mounting sheet 10 is fixed to a shield 9 inside the helmet 1.
Fig. 3 is a diagram exemplifying a state before mounting of the mounting sheet 10.
Fig. 4 is a schematic diagram for explaining a state where the mounting sheet 10 is mounted to the shield 9.
Fig. 5 is a diagram exemplifying a state where water 30 adheres to a surface of the mounting sheet 10 mounted to the shield 9, which corresponds to an inner side of the helmet.
Fig. 6 is a diagram exemplifying a state where the water 30 adheres between the shield 9 and the mounting sheet 10.
Fig. 7 is a diagram exemplifying a state (left end region or right end region) in which the mounting sheet 10 is mounted to the shield 9.
Fig. 8 is a flowchart of a method for mounting the mounting sheet 10 (S10).
Fig. 9 is a diagram exemplifying a state where the mounting sheet 10 is mounted to the inside of goggles 2 in Modification 1.
Fig. 10 is a diagram exemplifying mounting of the mounting sheet 10 in Modification 2.
Fig. 11 is a table and graph illustrating test results obtained by measuring an amount of deformation under humidification.
Fig. 12 is a photographed image showing an embodiment of a humidification deformation test.
Fig. 13 is a table illustrating test results obtained by measuring the deformation amount after long-term mounting to a visor shield and a photographed image showing the test results.
Fig. 14 is a table and graph illustrating test results obtained by measuring a haze under humidification.
Fig. 15 is a table and graph illustrating test results obtained by measuring surface hardness during dry or wet conditions.
Fig. 16 is a table and graph illustrating test results obtained by measuring a dropped sand surface abrasion value (HAZE value) during dry conditions.
Fig. 17 is a diagram exemplifying the mounting sheet 10 in Modification 3 and a method for mounting the mounting sheet 10.

### DESCRIPTION OF EMBODIMENTS

Hereinafter, embodiments of the present invention will be described with reference to the drawings.

First, the outline of this embodiment will be described.

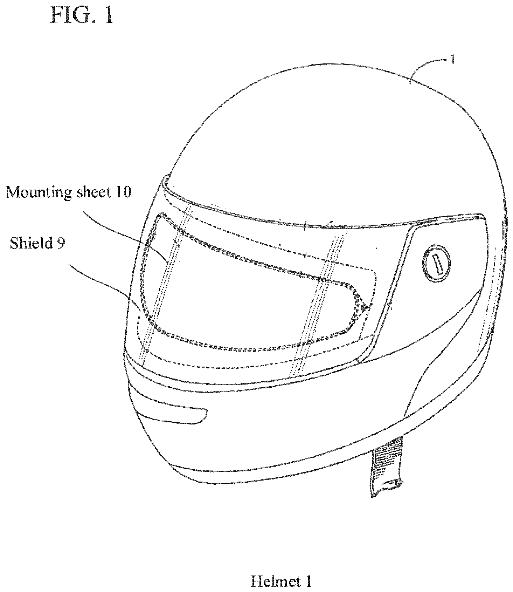

Fig. 1 is a diagram exemplifying an overall configuration of a helmet 1.

As exemplified in Fig. 1, the helmet 1 is provided with a visor shield 9 (hereinafter referred to as the shield 9), and a mounting sheet 10 is applied onto an inner wall surface of the shield 9 which is an inner side of the helmet 1.

The shield 9 is formed of a transparent resin. The shield 9 of this example is formed of, for example, polycarbonate.

The mounting sheet 10 is disposed in a region of the shield 9 including the viewing area of a wearer.

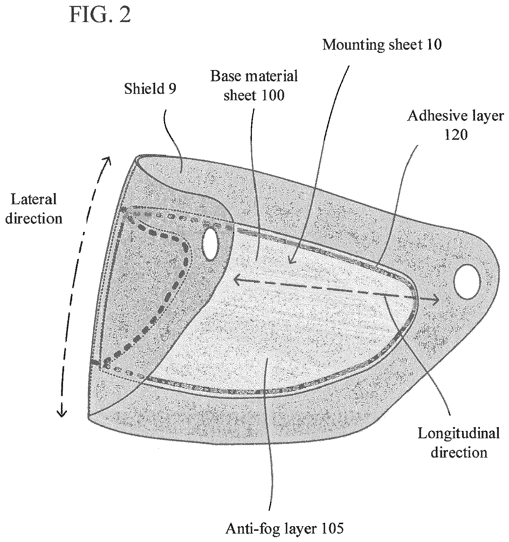

Fig. 2 is a diagram schematically representing a state where the mounting sheet 10 is fixed to the shield 9 inside the helmet 1.

As exemplified in Fig. 2, the mounting sheet 10 is mounted to the inside of the shield 9. In the shield 9, an inner wall surface corresponding to the inner side of the shield 9 is formed in a smooth three-dimensional concave curved surface shape, and the mounting sheet 10 is mounted in a deflected state along the curved surface with respect to the inner wall surface formed in the three-dimensional concave curved surface shape.

The mounting sheet 10 is configured to include a base material sheet 100 and an adhesive layer 120. In the mounting sheet 10, the adhesive layer 120 is disposed on one of both surfaces of the base material sheet 100, which faces the concave curved surface of the shield 9. Further, in the mounting sheet 10, the adhesive layer 120 is continuously disposed along an outer edge portion of the base material sheet 100. The mounting sheet 10 is mounted to the shield 9 in a state where a space between the shield 9 and a mounting sheet 10 is made to have airtightness by the continuously disposed adhesive layer 120. Further, in the mounting sheet 10, an anti-fog layer 105 is provided at least on the one of both surfaces of the base material sheet 100, which does not face the inner wall surface of the shield 9. The mounting sheet 10 is mounted to the shield 9 to function as an anti-fog sheet.

Next, a configuration of the mounting sheet 10 will be described.

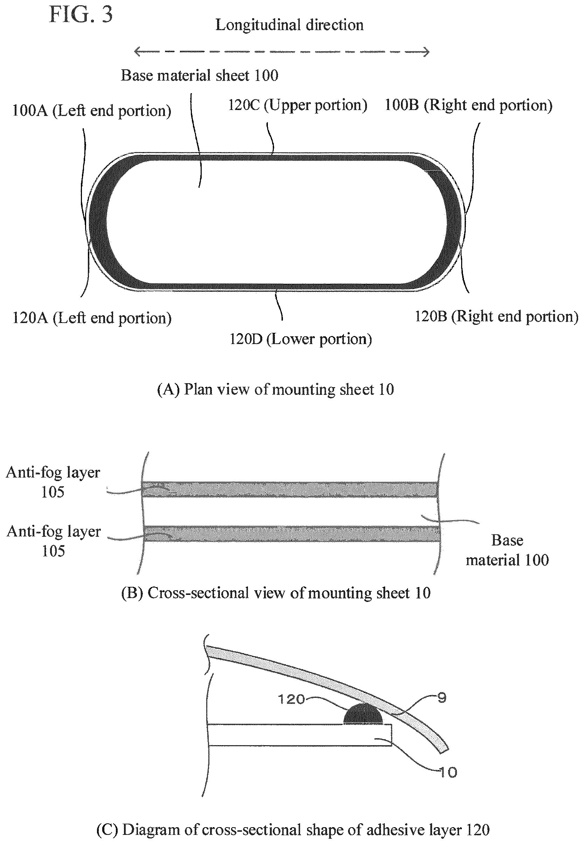

Fig. 3 is a diagram exemplifying a state before mounting of the mounting sheet 10.

Fig. 3(A) is a plan view exemplifying the mounting sheet 10, and Fig. 3(B) is a diagram exemplifying a cross section of the mounting sheet 10. Fig. 3(C) is a schematic diagram for explaining a cross-sectional shape of the adhesive layer 120.

As exemplified in Figs. 3(A) and 3(B), the mounting sheet 10 is configured to include the base material sheet 100, the anti-fog layer 105, and the adhesive layer 120.

In the mounting sheet 10, the anti-fog layer 105 is provided at least on the one of both surfaces of the base material sheet 100, which faces the inner wall surface of the shield 9.

In the mounting sheet 10, the anti-fog layer 105 may be formed on a surface of the base material sheet 100, or the base material sheet 100 and a transparent sheet on which the anti-fog layer 105 is formed may be bonded to each other to form the mounting sheet 10 as a single sheet. The mounting sheet 10 of this example will be described as a specific example in the case where the anti-fog layer 105 is formed on the surface of the base material sheet 100.

### [Base Material Sheet 100]

The base material sheet 100 is an example of a substrate according to the present invention.

The base material sheet 100 is a transparent sheet formed of a plate-shaped or sheet-shaped translucent resin. The base material sheet 100 is a sheet material having a curvature smaller than that of the inner wall surface of the shield 9 in a state where the base material sheet 100 is not mounted to the inner wall surface of the shield 9 and, the anti-fog layer 105 is provided on at least one of both surfaces of the base material sheet 100. The base material sheet 100 of this example is a flexible planar transparent sheet.

According to the invention, the base material sheet 100 is formed of polycarbonate, but in principle it would also be possible to form the base material sheet 100 of polyurethane, polyamide, or polyethylene terephthalate having permeability. In the described example, the base material sheet 100 is a transparent sheet formed of polycarbonate which is the same material as the shield 9. Since the base material sheet 100 is formed of polycarbonate, the base material sheet 100 has heat resistance and moisture absorption resistance. Since the base material sheet 100 has heat resistance and moisture absorption resistance, the base material sheet 100 is hardly affected by heat and moisture, so that the amount of deformation is small. Further, since the base material sheet 100 is formed of polycarbonate, the base material sheet 100 has flexibility and, at the same time, has a high repulsive force to restore the state before bending even if the base material sheet 100 is elastically bent. Since the base material sheet 100 can be elastically bent, the base material sheet 100 can be mounted in a deflected state to the inner wall surface of the shield 9. Since the base material sheet 100 has a high repulsive force, the base material sheet 100 can press the inner wall surface of the shield 9 by being mounted in a deflected state to the inner wall surface of the shield 9. Consequently, the base material sheet 100 is hardly affected by the adhesive strength of the adhesive layer 120.

The base material sheet 100 is a transparent sheet formed of polycarbonate and having a thickness of 0.1 mm or more. According to the claimed invention, the thickness of the base material sheet 100 is 0.2 mm or more and 1.0 mm or less. Preferably, the thickness of the base material sheet 100 is 0.2 mm or more and 0.6 mm or less. When the thickness of the base material sheet 100 is 0.2 mm or more and 0.6 mm or less, the base material sheet 100 can be deflected along the inner wall surface of the shield 9, which is a three-dimensional concave curved surface, and, at the same time, the base material sheet 100 can have flexibility enough not to be in contact with the inner wall surface of the shield 9 when mounted in a deflected state to the shield 9.

When the thickness of the base material sheet 100 is too small (less than 0.1 mm), the base material sheet 100 may be bent by the anti-fog layer 105 provided on the base material sheet 100, and therefore, the thickness should be 0.1 mm or more. The base material sheet 100 may have a thickness of 0.1 mm or more by applying a plurality of polycarbonate layers onto each other.

It is preferable that the thickness of the base material sheet 100 be selected according to the shape of the shield 9. Specifically, the thickness of the base material sheet 100 is preferably 0.2 mm to 0.5 mm when the shape of the inner wall surface of the shield 9 is a three-dimensional shape (such as a spherical or aspheric shape). Further, the thickness of the base material sheet 100 is preferably 0.5 mm to 1.0 mm when the shape of the inner wall surface of the shield 9 is a two-dimensional shape (such as a cylinder shape). In principle, the base material sheet 100 may have a thickness of 0.9 mm to 5.0 mm as long as it is premised that a plate-shaped polycarbonate is thermally bent with respect to the mounting sheet 10.

According to the claimed invention, the thickness of the mounting sheet 10 (that is, the total thickness of the base material sheet 100 and the anti-fog layer 105) is 5.0 mm or less. Preferably, the total thickness of the base material sheet 100 and the anti-fog layer 105 is 1.0 mm or less. More preferably, the thickness is 0.6 mm or less.

When the total thickness of the base material sheet 100 and the anti-fog layer 105 is 5.0 mm or more, it is difficult for the mounting sheet 10 to be deflected along the shape of the concave curved surface which is the inner wall surface of the shield 9, and the mounting sheet 10 may be peeled off from the shield 9. Thus, the total thickness of the base material sheet 100 and the anti-fog layer 105 is preferably 5.0 mm or less.

### [Anti-Fog Layer 105]

In accordance with the claimed invention, the anti-fog layer 105 is provided at least on the one of both surfaces of the base material sheet 100, which does not face the inner wall surface of the shield 9 when mounted to the inner wall surface of the shield 9. That is, the anti-fog layer 105 can be provided on a surface not facing the inner wall surface of the shield 9 or on both surfaces of the base material sheet 100. Fig. 3(B) shows an embodiment, where the anti-fog layers 105 are provided on both surfaces of the base material sheet 100.

The anti-fog layer 105 is a hydrophilic film chemically bonded to the surface of the base material sheet 100, specifically, a hydrophilic polymer or a hydrophilic anti-fog film formed of an anti-fog liquid mainly composed of a surfactant. The anti-fog layer 105 is formed, for example, by applying the anti-fog liquid onto the surface of the base material sheet 100 and drying the liquid.

In the anti-fog layer 105, the film thickness can be appropriately changed within the range to the extent that the base material sheet 100 formed of polycarbonate is not deformed.

The anti-fog layer 105 may be formed by applying an anti-fog liquid onto a surface of a transparent sheet mainly composed of polyethylene terephthalate (PET) or cellulose acetate, and the transparent sheet having the anti-fog layer 105 formed thereon and the base material sheet 100 formed of polycarbonate may be bonded to each other.

### [Adhesive Layer 120]

The adhesive layer 120 is an example of fixing means according to the present invention.

As exemplified in Fig. 3(A), the base material sheet 100 and the adhesive layer 120 are symmetrical. The adhesive layer 120 is continuously disposed along the outer edge portion of the base material sheet 100. Here, the term "continuously disposed" is a concept including not only a case where the adhesive layer 120 is continued in a straight line or a curved line as shown in Fig. 3(A) but also a broken line shape and the like. However, when the space between the shield 9 and the mounting sheet 10 is required to have airtightness, it is preferable that the adhesive layer 120 be in a closed state in a linear shape or a curved shape. In addition, the term "adhesion" in the present application means possession of adhesiveness and is a concept including adhesion due to only a pure adhesive and, in addition, adhesion in a state having viscosity before solidification of an adhesive or the like. Accordingly, the "adhesive" in the present application is a concept including not only a pure pressure-sensitive adhesive but also a viscous adhesive. In this embodiment, a pressure-sensitive adhesive, which does not solidify like an adhesive, will be described as a specific example. A pressure-sensitive adhesive which does not solidify is freely removable and superior in that it can be reattached.

The adhesive layer 120 is formed, for example, by applying a liquid elastic pressure-sensitive adhesive to the outer edge portion of the base material sheet 100 and drying the adhesive. That is, the adhesive layer 120 is formed of an elastic pressure-sensitive adhesive which has adhesiveness on its surface and can be elastically deformed with a predetermined volume, and has a function as an elastically deformable spacer and a function as a removable pressure-sensitive adhesive. A liquid elastic pressure-sensitive adhesive is, for example, a one-component moisture-reactive elastic pressure-sensitive adhesive such as CEMEDINE BBX 909 manufactured by Cemedine Co., Ltd. The adhesive layer 120 also functions as a sealing material for sealing the space between the base material sheet 100 and the shield 9, and since this sealing material itself has adhesiveness, high sealing performance can be expected.

As exemplified in Fig. 3(A), in the adhesive layer 120, the adhesive layer 120 at both end portions in a longitudinal direction of the base material sheet 100 (an adhesive layer left end portion 120A and an adhesive layer right end portion 120B) is formed as an elastic adhesive layer having a larger width than the adhesive layer 120 at a central portion in the longitudinal direction of the base material sheet 100 (an adhesive layer upper portion 120C and an adhesive layer lower portion 120D). Specifically, the adhesive layers 120A and 120B provided at a left end portion 100A and a right end portion 100B of the base material sheet 100 have a larger width than the adhesive layer 120C or 120D provided at upper or lower portion of the base material sheet 100. That is, the adhesive layers 120A and 120B provided at the left end portion and the right end portion of the base material sheet 100 have a stronger adhesive force than the adhesive layer 120C or 120D provided at the upper or lower portion of the base material sheet 100.

Further, as exemplified in Fig. 3(C), the adhesive layer 120 is raised so as to have a convex curved cross section. Since the adhesive layer 120 is formed of an elastic pressure-sensitive adhesive having the raised convex curved cross section, the adhesive layer 120 is elastically deformed to form a wide contact surface even when pressed at various abutment angles and can be adhered to an inner surface of the shield 9 at this contact surface.

Further, in the adhesive layer 120, if the adhesive layer 120 provided at the left and right end portions of the base material sheet 100 has a stronger adhesive force than the adhesive layer 120C or 120D provided at the upper or lower portion of the base material sheet 100, the adhesive layer 120 may be formed, for example, as an elastic adhesive layer in which the adhesive layer 120 at the both end portions in the longitudinal direction of the base material sheet 100 is raised higher than the adhesive layer 120 at the central portion in the longitudinal direction of the base material sheet 100.

As described above, the mounting sheet 10 is configured to include the base material sheet 100, the anti-fog layer 105, and the adhesive layer 120.

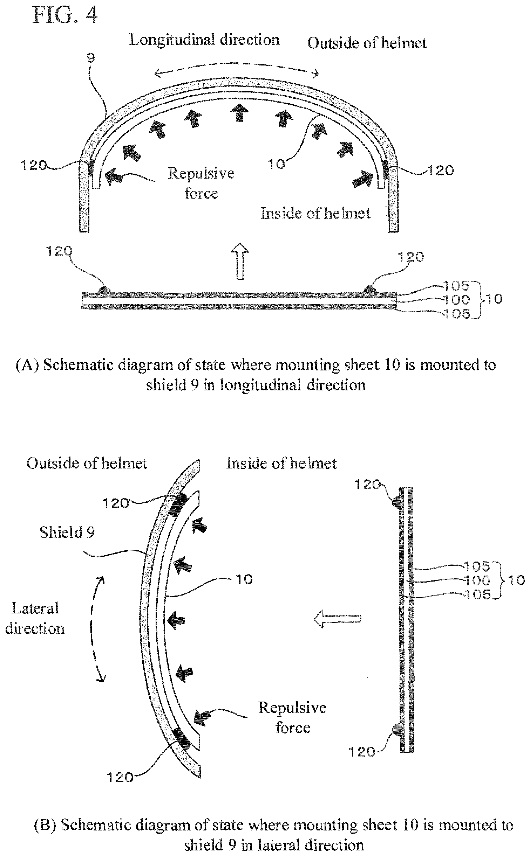

Next, operation of the mounting sheet 10 will be described with reference to Figs. 4 to 7.

Fig. 4 is a schematic diagram for explaining a state where the mounting sheet 10 is mounted to the shield 9.

Fig. 4(A) is a schematic diagram for explaining a state where the mounting sheet 10 is mounted to the shield 9 in the longitudinal direction, and Fig. 4(B) is a schematic diagram for explaining a state where the mounting sheet 10 is mounted to the shield 9 in the lateral direction.

As exemplified in Fig. 4(A), in the mounting sheet 10, the mounting sheet 10 having a substantially planar shape before mounting is deflected in the longitudinal direction to be curved and is mounted in the deflected state to the inner wall surface of the shield 9. Since the mounting sheet 10 is formed of polycarbonate, the mounting sheet 10 is elastically bent by being deflected. Thus, when the mounting sheet 10 is mounted to the shield 9, the force that deflects the mounting sheet 10 in the longitudinal direction is removed, whereby the mounting sheet 10 is to be restored to the state before mounting due to the repulsive force which is the force to restore the state before mounting. In the mounting sheet 10, due to this repulsive force, the adhesive layer 120 (not shown) continuous to the entire region of the outer edge portion of the base material sheet 100 is in entire contact with the inner wall surface of the shield 9 and can be adhesively fixed.

As exemplified in Fig. 4(B), the mounting sheet 10 is deflected in the longitudinal direction to be curved and is mounted in the deflected state to the inner wall surface of the shield 9, whereby the mounting sheet 10 is elastically bent in conformity with the curved surface of the inner wall surface of the shield 9 also in the lateral direction. When mounted to the shield 9, the mounting sheet 10 is to be restored to the state before mounting due to the repulsive force of the mounting sheet 10, and therefore, due to the repulsive force thereof, the adhesive layer 120 (not shown) continuous to the entire region of the outer edge portion of the base material sheet 100 is in entire contact with the inner wall surface of the shield 9 and can be adhesively fixed.

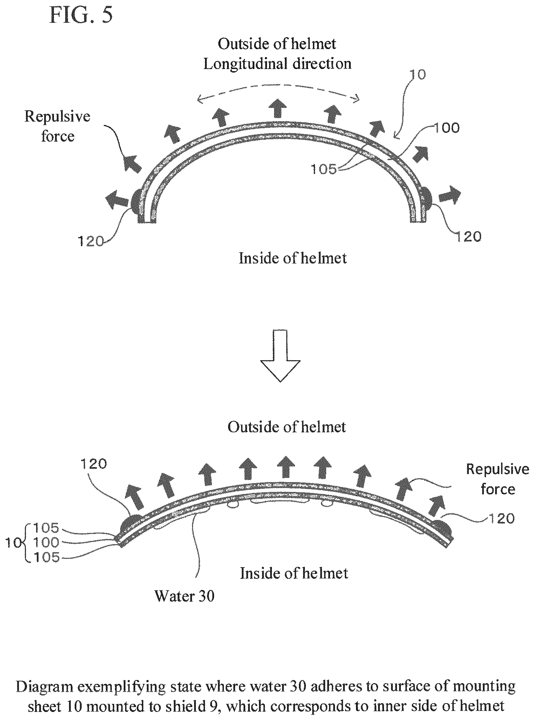

Fig. 5 is a diagram exemplifying a state where water 30 adheres to a surface of the mounting sheet 10 mounted to the shield 9, which corresponds to an inner side of a helmet.

As exemplified in Fig. 5, the mounting sheet 10 is mounted to the inner wall surface of the shield 9 (not shown) by the repulsive force of the base material sheet 100 and the adhesive force of the adhesive layer 120. In the mounting sheet 10, when a temperature difference occurs between the outside of a helmet 1 and the inside of the helmet 1, the water 30 adheres to one of both surfaces of the mounting sheet 10, which does not face the shield 9.

Inside the helmet 1, the mounting sheet 10 prevents fogging due to adhesion of the water 30 due to the anti-fog layer 105 provided on the surface of the base material sheet 100. In the mounting sheet 10, the water 30 adhered to the anti-fog layer 105 is absorbed by the anti-fog layer 105, and the base material sheet 100 does not absorb moisture, so that the repulsive force of the mounting sheet 10 is maintained.

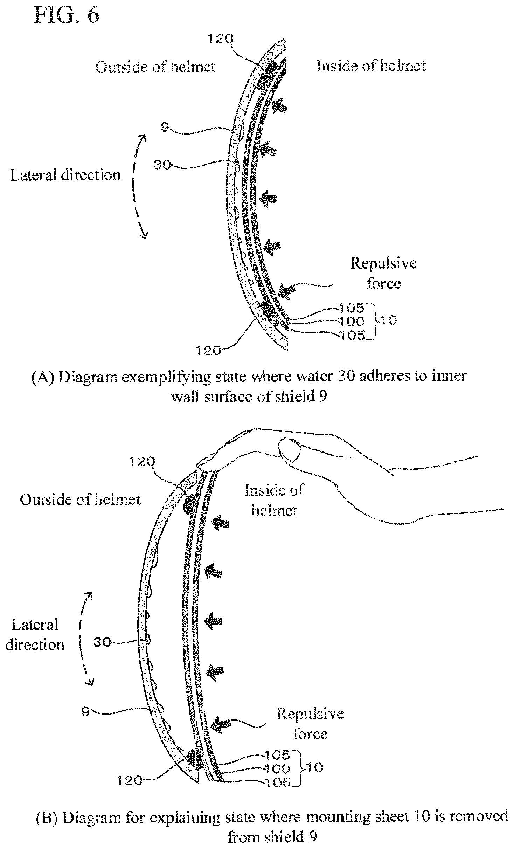

Fig. 6 is a diagram exemplifying a state where the water 30 adheres between the shield 9 and the mounting sheet 10.

Fig. 6(A) is a diagram exemplifying a state where the water 30 adheres to the inside of the shield 9, and Fig. 6(B) is a diagram for explaining a state of removing the mounting sheet 10 from the inner wall surface of the shield 9.

As exemplified in Fig. 6(A), the mounting sheet 10 is mounted to the inner wall surface of the shield 9 by the repulsive force of the base material sheet 100 and the adhesive force of the adhesive layer 120.

In the shield 9, due to the temperature difference between the outside of the helmet 1 and the inside of the helmet 1, the water 30 adheres to the inner wall surface of the shield 9 between the shield 9 and the mounting sheet 10.

As exemplified in Fig. 6(B), a wearer removes the mounting sheet 10 from the inner wall surface of the shield 9 in order to remove the water 30 on the inner wall surface of the shield 9.

Since the water 30 is present between the shield 9 and the mounting sheet 10, in the mounting sheet 10 the anti-fog layer 105 provided on the surface facing the inner wall surface of the shield 9 absorbs a moisture of the water 30.

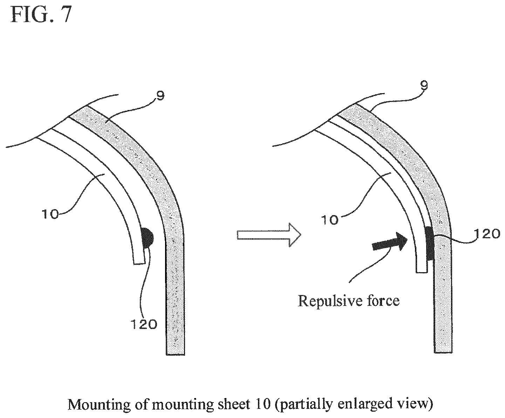

Fig. 7 is a diagram exemplifying a state (left end region or right end region) in which the mounting sheet 10 is mounted to the shield 9.

As exemplified in Fig. 7, the mounting sheet 10 is mounted to the inner wall surface of the shield 9 in a state of being deflected in the longitudinal direction. When the mounting sheet 10 is mounted to the shield 9, the force that deflects the mounting sheet 10 in the longitudinal direction is removed, and the repulsive force which is the force to restore the state before mounting is generated. In the mounting sheet 10, the left end region or the right end region of the mounting sheet 10 is pressed toward the inner wall surface of the shield 9 by the generated repulsive force. When the left end region or the right end region of the mounting sheet 10 is pressed toward the inner wall surface of the shield 9, the adhesive layer 120 disposed in a convex curved surface shape is deformed, is in entire contact with the inner wall surface of the shield 9, and can be adhesively fixed.

Next, a method for mounting the mounting sheet 10 will be described.

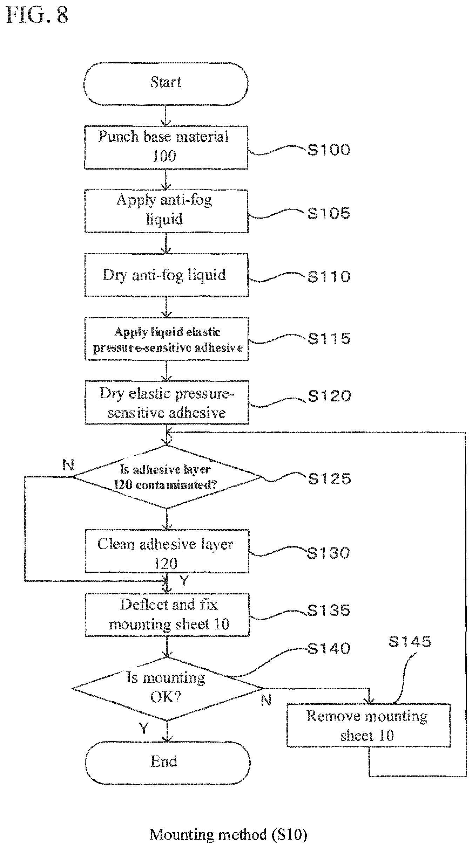

Fig. 8 is a flowchart of the method for mounting the mounting sheet 10 (S10).

As exemplified in Fig. 8, in step 100 (S100), a processing person punches the base material sheet 100 into a previously designed shape from a sheet of polycarbonate. The base material sheet 100 has a thickness of 0.1 mm or more.

In step 105 (S105), the processing person forms the anti-fog layer 105 on at least one of both surfaces of the punched base material sheet 100, which does not face the inner wall surface of the shield 9. In the mounting sheet 10 of this example, the anti-fog layers 105 are formed on the both surfaces of the base material sheet 100. For example, the processing person may apply an anti-fog liquid onto the surface of the base material sheet 100 using a roll coater, or may impregnate the base material sheet 100 with the anti-fog liquid and thereby apply the anti-fog liquid onto the surface of the base material sheet 100.

In step 110 (S110), the processing person dries the anti-fog liquid applied onto the base material sheet 100 to form the anti-fog layer 105 on the surface of the base material sheet 100.

In step 115 (S115), the processing person applies a liquid elastic pressure-sensitive adhesive to the outer edge portion of the base material sheet 100 on which the anti-fog layer 105 is formed. The elastic pressure-sensitive adhesive is applied, for example, by moving a nozzle, through which the pressure-sensitive adhesive is ejected, to a preprogrammed position at a preprogrammed speed. Consequently, the liquid elastic pressure-sensitive adhesive can be applied to have a desired shape, width and height.

In step 120 (S120), the processing person dries the elastic pressure-sensitive adhesive applied onto the base material sheet 100 to form the adhesive layer 120 on the base material sheet 100. The dried adhesive layer 120 may be covered with a protective sheet such as a film to prevent adhesion of dust or the like.

In step 125 (S125), the processing person checks whether the adhesive layer 120 is contaminated. If the adhesive layer 120 is contaminated, the flowchart proceeds to S130, and when the adhesive layer 120 is not contaminated, the flowchart proceeds to S135.

In step 130 (S130), the processing person washes the surface of the adhesive layer 120 with water. Adhesiveness of the surface of the adhesive layer 120 is restored by removing contamination such as dust by washing with water or the like.

In step 135 (S135), the processing person deflects the mounting sheet 10 in the longitudinal direction to curve the mounting sheet 10, and mounts the mounting sheet 10 in a deflected state to the inner wall surface of the shield 9. More specifically, the processing person mounts the mounting sheet 10 to the inner wall surface of the shield 9 in such a state that the surface of the mounting sheet 10, which does not face the inner wall surface of the shield 9 (the surface on which the adhesive layer 120 is not provided), is deflected inward. After mounting the mounting sheet 10 to the inner wall surface of the shield 9, the processing person presses the outer edge portion of the mounting sheet 10 toward the shield 9, so that the entire adhesive layer 120 is brought into contact with the inner wall surface of the shield 9.

In step 140 (S140), the processing person checks whether or not the entire adhesive layer 120 is sufficiently in contact with the inner wall surface of the shield 9 and adheres thereto. When a portion of the adhesive layer 120 is not sufficiently in contact with the inner surface of the shield 9, the flowchart proceeds to S145, and when the entire adhesive layer 120 is in contact with the inner surface of the shield 9, the mounting method (S10) is completed.

In step 145 (S145), the processing person removes the mounting sheet 10 from the inner wall surface of the shield 9 and starts over from S125.

In this manner, in the mounting method (S10) of the present embodiment, the mounting position of the mounting sheet 10 is determined while slightly changing the position of the mounting sheet 10.

As described above, in the helmet 1 of the present embodiment, the mounting sheet 10, which is configured to include the plate-shaped base material sheet 100, formed of polycarbonate and having a curvature smaller than that of the mounting surface when not mounted to the mounting surface, and the anti-fog layer 105 provided at least on one of the both surfaces of the base material sheet 100, which does not face the inner wall surface of the shield 9 when mounted to the inner wall surface of the shield 9, is deflected in the longitudinal direction to be mounted to the inner wall surface of the shield 9. In the helmet 1, the mounting sheet 10 is mounted in the deflected state to the inner wall surface of the shield 9, whereby the adhesive layer 120 continuously disposed along the entire region of the outer edge portion of the base material sheet 100 can be in entire contact with the inner wall surface of the shield 9 and pressed using the repulsive force to restore the state before mounting (deflection), so that the mounting sheet 10 can be prevented from peeling from the inner wall surface of the shield 9.

Further, in the helmet 1, the mounting sheet 10 can be removably fixed in such a state that the mounting sheet 10 is spaced apart from the shield 9 by the adhesive layer 120 and sealed. Consequently, a dew condensation prevention function can be added to the helmet. In particular, since the adhesive layer 120 has the convex curved cross section, the adhesive layer 120 can correspond to shields of various shapes.

The mounting sheet 10 of the present embodiment has a relatively simple manufacturing process, so that a general user can perform some operations (such as adjustment of a pasting position and pasting operation). Since the adhesive force of the adhesive layer 120 is restored to a certain extent by washing with water, maintenance can be easily performed.

### [Modification 1]

Next, a modification of the above embodiment will be described.

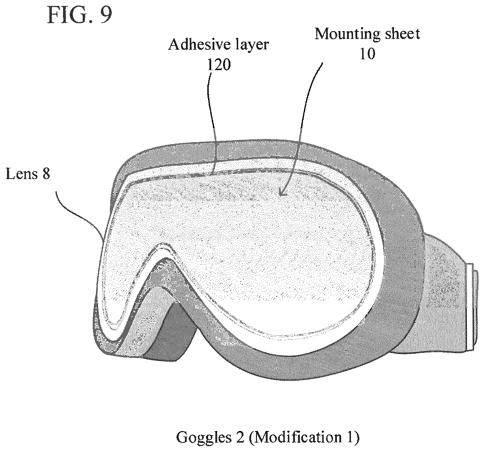

Fig. 9 is a diagram exemplifying a state where the mounting sheet 10 is mounted to the inside of goggles 2 in Modification 1.

As exemplified in Fig. 9, the mounting sheet 10 may be disposed on a goggle lens 8 (hereinafter referred to as the lens 8) of the goggles 2. The mounting sheet 10 is formed such that the base material sheet 100 matches the shape of the lens 8, and the anti-fog layer 105 (not shown) is provided on at least one of both surfaces of the base material sheet 100, which does not face an inner wall surface of the lens 8. In the mounting sheet 10, the adhesive layer 120 is continuously disposed along the outer edge portion of the base material sheet 100.

As described above, the mounting sheet 10 can also be used for the lens 8 of the goggles 2 and is removable, so that even when water adheres between an inner wall surface of the mounting sheet 10 and the lens 8, the mounting sheet 10 can be easily mounted after cleaning.

### [Modification 2]

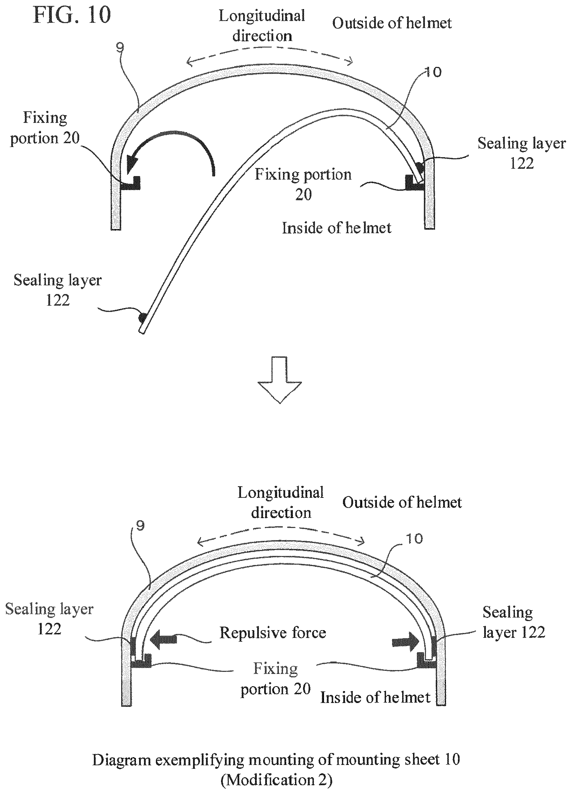

Fig. 10 is a diagram exemplifying mounting of the mounting sheet 10 in Modification 2.

As exemplified in Fig. 10, the shield 9 is provided with a fixing portion 20 on its inner wall surface. The fixing portion 20 is mounted to the inner wall surface of the shield 9 and is formed in, for example, a hook shape so that the end portion of the mounting sheet 10 can be fixed.

Further, the fixing portions 20 are provided at both ends of the mounting sheet 10 mounted to the inner wall surface of the shield 9. Specifically, the fixing portion 20 is disposed at a position of the inner wall surface of the shield 9 where at least each end portion of the mounting sheet 10 in the longitudinal direction can be sandwiched and fixed in a deflected state. Furthermore, the fixing portion 20 is disposed at a position where the mounting sheet 10 can be fixed to such an extent that a sealing layer 122 can be pressed against the inner wall surface of the shield 9 in a state where the mounting sheet 10 is fixed.

In the mounting sheet 10, the sealing layer 122 is continuously provided along the outer edge portion of the base material sheet 100. The sealing layer 122 is a liquid elastic sealing material, for example, silicone. Since the sealing layer 122 is continuously disposed, the sealing layer 122 can be mounted to the shield 9 in a state where a space between the shield 9 and the mounting sheet 10 has airtightness.

As exemplified in Fig. 10, one end portion of the mounting sheet 10 is hooked and fixed to one of the fixing portions 20 provided on the inner wall surface of the shield 9. The mounting sheet 10 is deflected in the longitudinal direction in a state where one of the end portions is fixed, and while the mounting sheet 10 is deflected, the other end portion of the mounting sheet 10 is hooked and fixed to the other fixing portion 20 provided on the inner wall surface of the shield 9.

In this manner, the mounting sheet 10 can be provided in a deflected state on the inner wall surface of the shield 9 by the fixing portion 20 provided on the inner wall surface of the shield 9. Since the mounting sheet 10 is disposed at a position, where the mounting sheet 10 can be fixed to such an extent that the sealing layer 122 can be pressed against the inner wall surface of the shield 9, by the fixing portion 20, the mounting sheet 10 can be configured even if the sealing layer 122 does not have adhesiveness.

Although the embodiment according to the present invention has been described above, the invention is not limited thereto, and various modifications, additions, and the like are possible without departing from the gist of the invention.

The helmet may not be used only for a motorcycle but may also be used as a helmet for disaster prevention or the like. The goggles may not be used only for goggles for skiing or snowboarding but may also be used as goggles for swimming and the like. The present invention may be applied to glasses.

### [Evaluation of Performance between the Present Example and Comparative Examples]

The performance of each anti-fog sheet of the present example, Comparative Example 1, and Comparative Example 2 was evaluated by the following tests.

First, the configurations of the present Example, Comparative Example 1, and Comparative Example 2 will be described.

The present Example is an anti-fog sheet formed of polycarbonate (PC). The anti-fog sheet of the present Example has a thickness of 0.3 mm and a transmittance of 89.89% (EN Calculation).

Comparative Example 1 is an anti-fog sheet formed of cellulose acetate (CA). The anti-fog sheet of Comparative Example 1 has a thickness of 0.8 mm and a transmittance of 86.24% (EN Calculation).

Comparative Example 2 is an anti-fog sheet formed of cellulose propionate (CP). The anti-fog sheet of Comparative Example 2 has a thickness of 0.8 mm and a transmittance of 86.79% (EN Calculation).

In the method for calculating the transmittance of each of the above anti-fog sheets, the transmittance is calculated by applying light from ultraviolet rays to infrared rays using a spectrometer.

### [1. Deformation Test by Humidification]

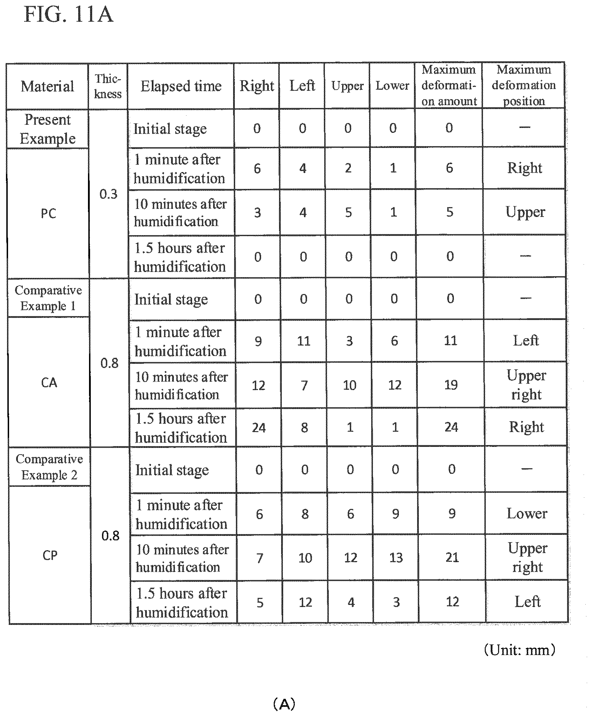

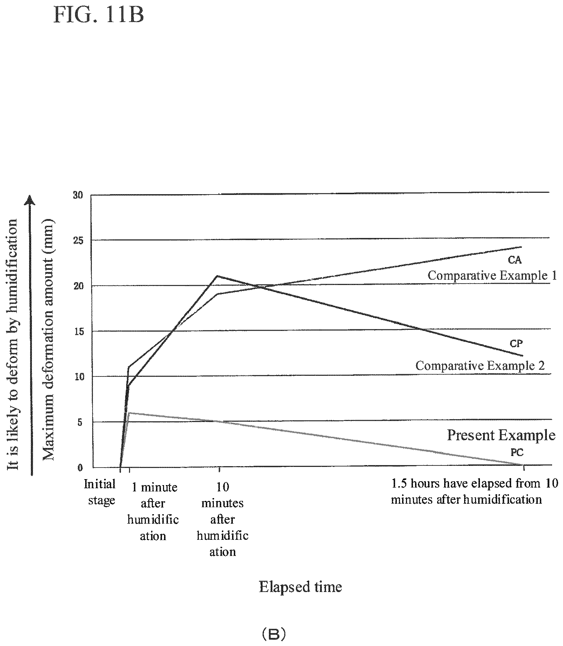

Fig. 11 is a table and graph illustrating test results obtained by measuring an amount of deformation under humidification, Fig. 11(A) is a table, and Fig. 11(B) is a graph.

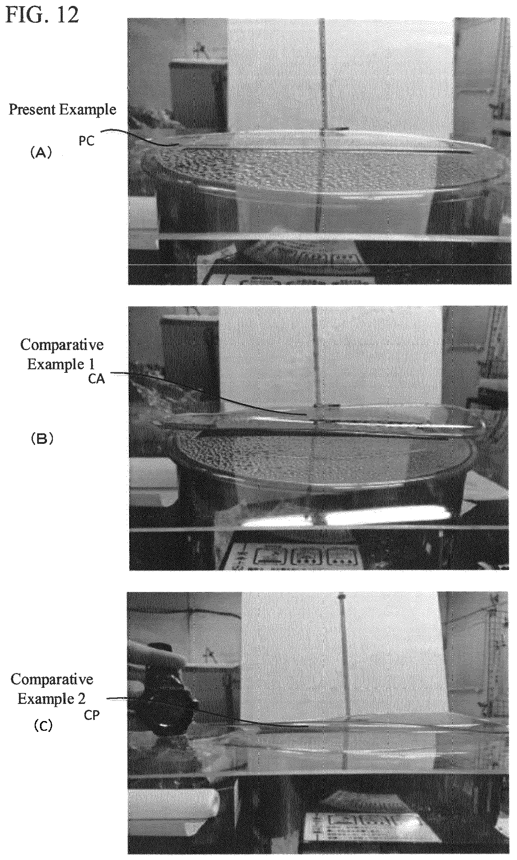

Fig. 12 is a photographed image showing an embodiment of a humidification deformation test. Fig. 12(A) is a diagram exemplifying an embodiment of the humidification deformation test of the present Example, Fig. 12(B) is a diagram exemplifying an embodiment of the humidification deformation test of Comparative Example 1, and Fig. 12(C) is a diagram exemplifying an embodiment of the humidification deformation test of Comparative Example 2.

### (Test Purpose)

For the purpose of the test, the deformation amounts obtained when humidifying the anti-fog sheets of the present Example, Comparative Example 1, and Comparative Example 2 are compared and evaluated.

### (Test Method)

As a test method, as exemplified in Fig. 12, a water bath of about 50°C is covered with an acrylic plate with a hole formed partially therethrough. An anti-fog sheet was placed at a position overlapping the hole provided in the acrylic plate, and one surface of the anti-fog sheet was humidified.

### (Test Results)

As exemplified in Fig. 11, the deformation amounts of the anti-fog sheets of the present Example, Comparative Example 1, and Comparative Example 2 were measured at respective times "initial stage (no humidification)", "1 minute after humidification", "10 minutes after humidification", and "after 1.5 hours have elapsed from 10 minutes after humidification".

Although deformation of the present Example was observed after humidification ("1 minute after humidification" and "10 minutes after humidification"), the deformation was zero "after 1.5 hours have elapsed from 10 minutes after humidification" (the state is similar to the "initial stage").

On the other hand, the anti-fog sheets of Comparative Example 1 and Comparative Example 2 were deformed to a greater extent than the anti-fog sheet of the present Example after humidification ("1 minute after humidification" and "10 minutes after humidification"). "After 1.5 hours have elapsed from 10 minutes after humidification", the deformation was not eliminated unlike the present Example, and each state was not restored (to the same state as the "initial stage").

The thickness of the anti-fog sheet of the present Example is 0.3 mm, whereas the thickness of each of the anti-fog sheets of Comparative Example 1 and Comparative Example 2 is as thick as 0.8 mm. Since Comparative Example 1 and Comparative Example 2 were thicker than the present Example, they should be originally advantageous for deformation; however, they were greatly deformed.

### [2. Plastic Deformation Test]

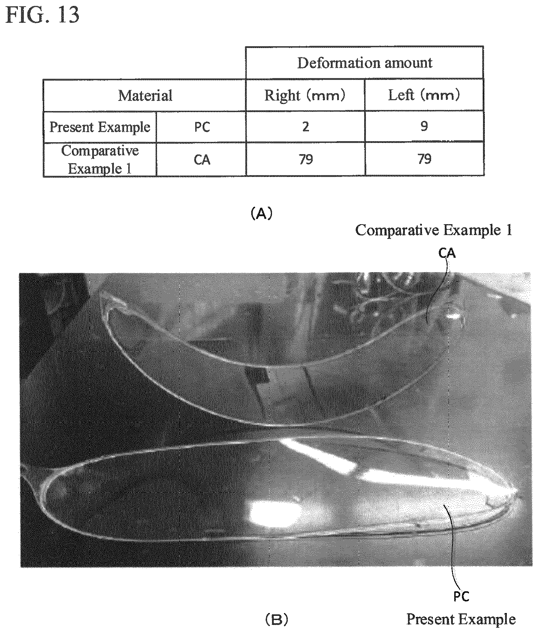

Fig. 13 is a table illustrating test results obtained by measuring the deformation amount after long-term mounting to a visor shield and a photographed image showing the test results. Fig. 13(A) is a table illustrating the test results, and Fig. 13(B) is a photographed image showing the test results.

### (Test Purpose)

For the purpose of the test, an anti-fog sheet is mounted to the visor shield for a long period of time and then removed, and the deformation amounts due to plastic deformation of the anti-fog sheets of the present Example, Comparative Example 1, and Comparative Example 2 were compared and evaluated.

### (Test Method)

The test method is as follows. A shield visor of the helmet manufacturer C is placed for 50 days under normal temperature and normal humidity in such a state that an anti-fog sheet is mounted to the shield visor, the anti-fog sheet is removed from the shield visor after 50 days, and the deformation amount of the anti-fog sheet is measured. The normal temperature is, for example, in the range of 5 to 35°C, and the normal humidity is, for example, in the range of 45 to 85%.

### (Test Results)

As exemplified in Fig. 13, in the present Example, even when the shape is kept constant for a long period of time, the shape is restored to substantially the same as the shape before mounting unless excessive heat is applied.

On the other hand, in Comparative Example 1, when the shape is kept constant for a long period of time, an arbitrary shape (the curved surface shape of the shield visor) is maintained and does not return to the original shape unless stress in the opposite direction to the deformation direction is applied.

### [3. Anti-fog Test]

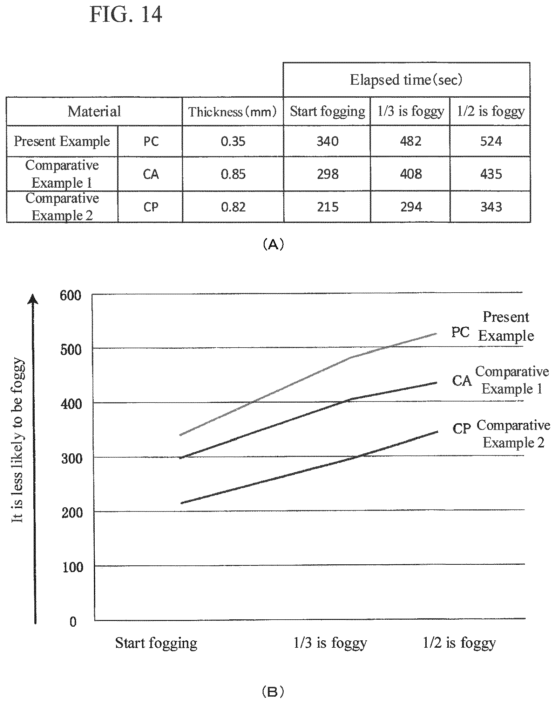

Fig. 14 is a table and graph illustrating test results obtained by measuring a haze under humidification, Fig. 14(A) is a table, and Fig. 14(B) is a graph.

### (Test Purpose)

For the purpose of the test, the anti-fog performances obtained when humidifying the anti-fog sheets of the present Example, Comparative Example 1, and Comparative Example 2 are compared and evaluated.

### (Test Method)

The test method was as follows. Each of the anti-fog sheets of the present Example, Comparative Example 1, and Comparative Example 2 was formed into a sheet piece of 30 × 40 mm, steam heated to a constant temperature was blown to the sheet piece at a constant flow rate, and "time to start fogging the sheet", "time until 1/3 of the sheet is foggy" per measurement area, and "time until half of the sheet is foggy" per measurement area were measured.

### (Test Results)

As exemplified in Fig. 14, the present Example had higher anti-fog performance than Comparative Example 1 and Comparative Example 2 formed of a hydrophilic material.

### [4. Surface Hardness Test 1]

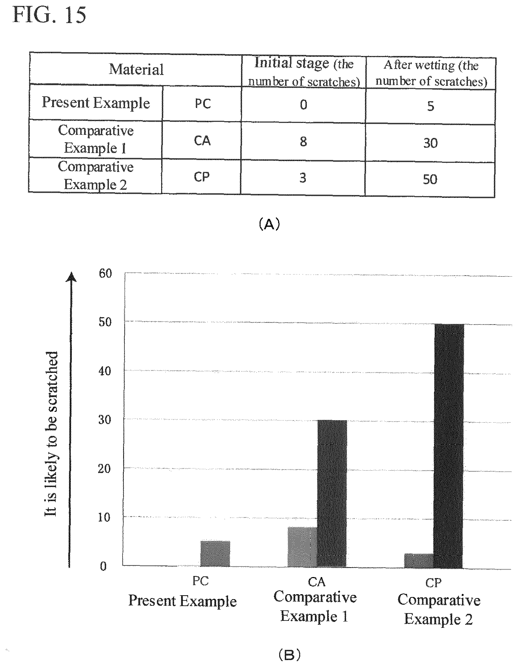

Fig. 15 is a table and graph illustrating test results obtained by measuring surface hardness during dry or wet conditions, Fig. 15(A) is a table, and Fig. 15(B) is a graph.

### (Test Purpose)

For the purpose of the test, the anti-fog surface wear resistance performances during dry or wet conditions of the anti-fog sheets of the present Example, Comparative Example 1, and Comparative Example 2 are compared and evaluated.

### (Test Method)

The test method is as follows. In a state where a specimen of the anti-fog sheet and a paper piece of a JK Wiper (registered trademark) are in contact with each other, they are rubbed relative to each other while applying a constant load (500 g) thereto and reciprocated in a certain direction an arbitrary number of times to be brought into friction with each other. The number of scratches formed on the surface of the specimen is measured by reciprocation in a certain direction and friction. In this test method, the same test is conducted under each of the dry and wet conditions. The dry condition (initial stage) is a state where moisture is not contained, and the wet condition (after wetting) is a state where a certain amount of moisture is contained in the surface.

### (Test Results)

As exemplified in Fig. 15, the sheet surface of the present Example is less likely to be scratched than Comparative Example 1 and Comparative Example 2, during both the dry and wet conditions. The reason for this result is that the anti-fog sheet of the present Example is formed of polycarbonate having moisture absorption resistance, and the surface thereof is provided with an anti-fog layer formed using antifogging processing. Consequently, in the present Example, even during the wet conditions, the surface hardness can be maintained without containing moisture, and the surface is hardly scratched.

On the other hand, in Comparative Example 1 and Comparative Example 2, the sheet surfaces are more likely to be scratched than the present example during the wet conditions. The reason for this result is that the anti-fog sheets of Comparative Example 1 and Comparative Example 2 are formed of hygroscopic materials. Thus, since the sheet surfaces of Comparative Example 1 and Comparative Example 2 contain moisture during the wet conditions, the surface hardness is less likely to be maintained, so that the surfaces are likely to be scratched.

### [5. Surface Hardness Test 2]

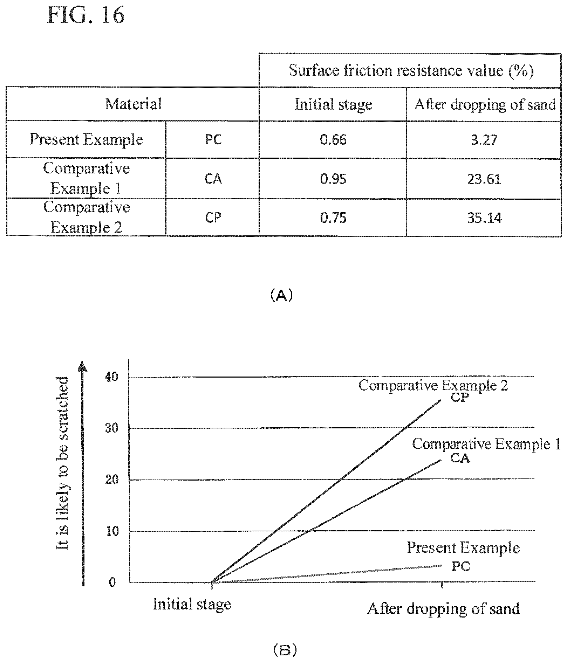

Fig. 16 is a table and graph illustrating test results obtained by measuring a dropped sand surface abrasion value (HAZE value) during dry conditions, Fig. 16(A) is a table, and Fig. 16(B) is a graph.

### (Test Purpose)

For the purpose of the test, the turbidities (HAZE values) during dry conditions of the anti-fog sheets of the present Example, Comparative Example 1, and Comparative Example 2 are compared and evaluated.

### (Test Method)

The test method is as follows. 400 g of sand (abrasive material or grinding material) having a prescribed particle diameter is dropped on an anti-fog surface which is the surface of a specimen of the anti-fog sheet, and the turbidity on the surface of the specimen after dropping of the sand is measured using a haze meter (see JIS T-8147: 2003 8.1e). This test method is performed under the dry conditions.

### (Test Results)

As exemplified in Fig. 16, at the initial stage (before dropping of sand), the present Example has a HAZE value that is not greatly different from those of Comparative Example 1 and Comparative Example 2.

On the other hand, after dropping of the sand, the HAZE value in the present Example was clearly lower than those in Comparative Example 1 and Comparative Example 2.

From the above test results, the anti-fog sheet of the present Example is less susceptible to heat and moisture and less likely to be scratched than the anti-fog sheets of Comparative Example 1 and Comparative Example 2. In addition, the anti-fog sheet of the present Example is less likely to be plastically deformed than the anti-fog sheets of Comparative Example 1 and Comparative Example 2, and after being mounted in a further deflected state to an inner wall surface of the visor shield, the anti-fog sheet of the present Example had a high restoring force to return to the shape before deflection.

In the present invention, the foregoing description of the illustrative embodiment is provided for the purpose of illustration and description, does not cover the scope of the invention, and is not intended to limit the disclosed invention. It is obvious for those skilled in the art that many modifications and variations are possible. The embodiment has been chosen and described in order to best describe the principles of the invention, and its practical applications to thereby enable those skilled in the art to understand the invention with various embodiments and modifications as is suited to the particular use contemplated. The scope of the invention is defined by the following claims.

### [Modification 3]

As described above, the mounting sheet 10 in which the substrate is formed of polycarbonate has the feature of maintaining elasticity and being difficult to deform under a high-humidity environment. Utilizing this feature, the following modification can also be proposed. That is, at least fixing means which restricts the movement of the mounting sheet 10 in the direction along the inner wall surface of the visor shield or the goggle lens is provided on the inner wall surface of the visor shield or the goggle lens, and at least the end portions of the mounting sheet 10 facing each other (for example, both ends in the longitudinal direction of the mounting sheet 10) are fixed by the fixing means so as not to spread in the direction along the inner wall surface. The fixing means may be an elastic adhesive layer or an adhesive seal provided on a periphery of the mounting sheet 10 or may be a plurality of protrusions protruding from the inner wall surface.

When the mounting sheet 10 is fixed by the fixing means in a state of being deflected along the inner wall surface of the visor shield or the goggle lens, even if the visor shield or the goggle lens is deformed to widen a distance between the plurality of fixing means, the engagement state between the fixing means and the mounting sheet 10 is maintained by the elastic force of the mounting sheet 10, and prevention of falling off of the mounting sheet 10 can be expected. In the mounting sheet 10 of the present Example, since deformation at the end portion of the mounting sheet 10 in a direction spaced apart from the inner wall surface of the visor shield or the goggle lens is smaller than that in the comparative examples even under a high-humidity environment, it suffices that only an outward direction along the inner wall surface is restricted as a restriction direction of the fixing means.

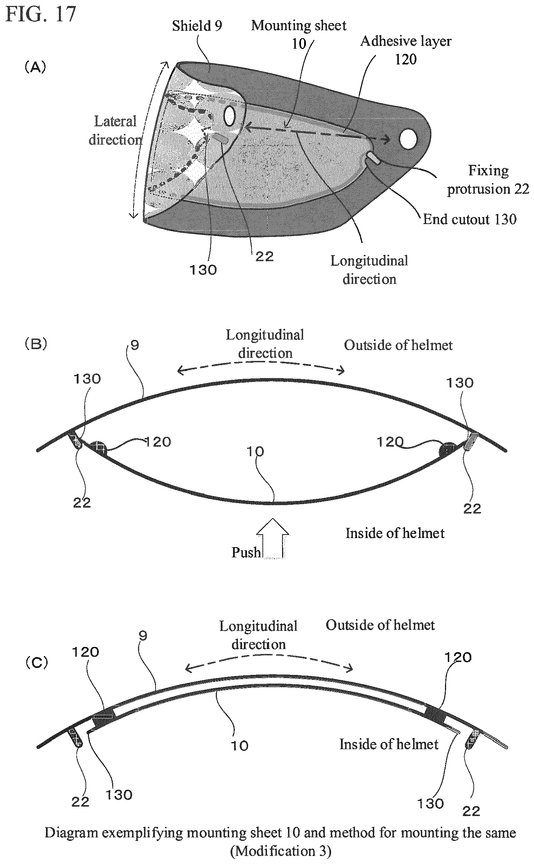

Fig. 17 is a diagram exemplifying the mounting sheet 10 in Modification 3 and a method for mounting the mounting sheet 10. In the present Example, the adhesive layer 120 is provided on the mounting sheet 10, but it is not indispensable.

As exemplified in Fig. 17(A), end cutouts 130 are provided at both ends in the longitudinal direction of the mounting sheet 10 of the present Example. In addition, two fixing protrusions 22 are provided on the inner wall surface of the shield 9 of the present Example. The distance between the two fixing protrusions 22 is a sufficient distance to engage the mounting sheet 10 in a state where the mounting sheet 10 is deflected along the inner wall surface of the shield 9, as exemplified in Fig. 17(A). The end cutout 130 may be in contact with or slightly spaced apart from the fixing protrusion 22.

In the mounting method in the present Example, as shown in Fig. 17(B), first of all, while the mounting sheet 10 is deflected, the end portions of the mounting sheet 10 facing each other (end cutouts 130) are engaged with the two fixing protrusions 22 provided on the inner wall surface of the shield 9. Then, as exemplified in Fig. 17(C), the mounting sheet 10 is pressed against the inner wall surface of the shield 9 while the end portions (end cutouts 130) are engaged.

When the adhesive layer 120 or an adhesive tape which is an elastic adhesive layer is provided at the outer edge portion of the mounting sheet 10, the mounting sheet 10 is firmly pressed against the inner wall surface of the shield 9 and adhered thereto. When the adhesive layer 120 or the adhesive tape is not provided at the outer edge portion of the mounting sheet 10, the fixing protrusions 22 on the inner wall surface of the shield 9 are firmly fitted into the end cutouts 130 of the mounting sheet 10.

As described above, in this modification, the fixing projection 22 is used as a positioning mechanism for determining a mounting position of the mounting sheet 10.

### REFERENCE SIGNS LIST

- 1: helmet
- 9: shield
- 10: mounting sheet
- 100: base material sheet
- 105: anti-fog layer
- 120: adhesive layer
- 2: goggles
- 8: lens
- 20: fixing portion
- 122: sealing layer
- 130: end cutout
- 22: fixing protrusion

## Claims

1. A mounting sheet (10) to be mounted to a concave curved inner wall surface of a visor shield (9) or a goggle lens (8) in a state where the mounting sheet (10) is deflected along the curved surface, comprising:
a base material sheet (100) which has a curvature smaller than that of the concave curved inner wall surface of the visor shield (9) or the goggle lens (8) when not mounted to the concave curved inner wall surface of the visor shield (9) or the goggle lens (8); and
**characterized in that**
the base material sheet (100) is formed of polycarbonate has a thickness of 0.2 mm or more and 1.0 mm or less; and
the mounting sheet (10) further comprises an anti-fog layer (105) provided at least on the one of both surface of the base material sheet (100), which does not face the concave curved inner wall surface of the visor shield (9) or the goggle lens (8) when mounted to the concave curved inner wall surface of the visor shield (9) or the goggle lens (8),
wherein a total thickness of the base material sheet (100) and the anti-fog layer (105) is 5.0 mm or less.

2. The mounting sheet (10) according to claim 1, wherein the concave curved inner wall surface of the visor shield (9) or the goggle lens (8) is a smooth three-dimensional concave curved inner wall surface of the visor shield (9) or the goggle lens (8), and the thickness of the base material sheet (100) is 0.2 mm or more and 0.6 mm or less.

3. The mounting sheet (10) according to claim 1 or 2, wherein the anti-fog layer (105) is a hydrophilic anti-fog film chemically bonded to said one surface of the base material sheet (100).

4. A helmet (1) comprising:
a visor shield (9);
a mounting sheet (10) to be mounted to a concave curved inner wall surface of the visor shield (9); and
fixing means (20, 120) for fixing the mounting sheet (10) to the concave curved inner wall surface of the visor shield (9);
wherein the mounting sheet (10) is a mounting sheet (10) according to one of claims 1 to 3; and
wherein the fixing means (20, 120) is at least provided at both ends in a longitudinal direction of the mounting sheet (10) and fixes the mounting sheet (10) to the visor shield (9) at the both ends.

5. The helmet according to claim 4, wherein the fixing means is an elastic adhesive layer (120) disposed between the visor shield (9) and the mounting sheet (10) and having a convex curved surface on a surface facing at least one of the visor shield (9) and the mounting sheet (10).

6. The helmet according to claim 4, wherein the visor shield (9) is formed of polycarbonate, and the fixing means (20, 120) sandwiches and fixes both ends of the mounting sheet (10) in a deflected state.

7. The helmet according to claim 6, wherein the fixing means is at least an elastic adhesive layer (120) continuously provided along an outer edge portion of the mounting sheet (10).

8. The helmet according to claim 7, wherein the fixing means is an elastic adhesive layer (120) which is highly raised or has a large width at both longitudinal end portions of the mounting sheet (10), rather than at a longitudinal center portion of the mounting sheet (10).

9. Goggles comprising:
a goggle lens (8);
a mounting sheet (10) to be mounted to a concave curved inner wall surface of the goggle lens (8); and
fixing means (20, 120) for fixing the mounting sheet (10) to the concave curved inner wall surface of the goggle lens (8);
wherein the mounting sheet (10) is a mounting sheet (10) according to one of claims 1 to 3; and
wherein the fixing means (20, 120) is at least provided at both ends in a longitudinal direction of the mounting sheet (10) and fixes the mounting sheet (10) to the goggle lens (8) at the both ends.

10. A method for mounting an anti-fog sheet (105) comprising the steps of:
forming an anti-fog layer (105) on at least one surface of a polycarbonate base material sheet (100) having a thickness of 0.2 mm or more and 1.0 mm or less; and
fixing the base material sheet (100) to a concave curved inner wall surface of a visor shield (9) or the goggle lens (8) in a state where the base material sheet (100) is deflected such that the surface formed with the anti-fog layer (105) is a concave curved surface;
wherein a total thickness of the base material sheet (100) and the anti-fog layer (105) is 5.0 mm or less.

## Patentansprüche

1. Befestigungsfolie (10) zum befestigen auf einer konkaven gekrümmten Innenwandfläche eines Visierschilds (9) oder eines Brillenglases (8) in einem Zustand, in dem die Befestigungsfolie (10) entlang der gekrümmten Oberfläche durchgebogen ist, umfassend:
eine Grundmaterialfolie (100), die eine Krümmung aufweist, welche kleiner als die der konkaven gekrümmten Innenwandfläche des Visierschilds (9) oder des Brillenglases (8) ist, wenn sie nicht auf der konkaven gekrümmten Innenwandfläche des Visierschilds (9) oder des Brillenglases (8) angebracht ist; und
**dadurch gekennzeichnet, dass**
die Grundmaterialfolie (100) aus Polycarbonat ausgebildet ist und eine Stärke von 0,2 mm oder mehr und 1,0 mm oder weniger aufweist; und
die Befestigungsfolie (10) ferner eine Antibeschlagsschicht (105) umfasst, die zumindest auf der einen von beiden Oberflächen der Grundmaterialfolie (100) vorgesehen ist und der konkaven gekrümmten Innenwandfläche des Visierschilds (9) oder des Brillenglases (8) nicht zugekehrt ist, wenn sie an der konkaven gekrümmten Innenwandfläche des Visierschilds (9) oder des Brillenglases (8) angebracht ist,
wobei eine Gesamtstärke der Grundmaterialfolie (100) und der Antibeschlagsschicht (105) 5,0 mm oder weniger beträgt.

2. Befestigungsfolie (10) nach Anspruch 1, wobei die konkave gekrümmte Innenwandfläche des Visierschilds (9) oder des Brillenglases (8) eine glatte dreidimensionale konkave gekrümmte Innenwandfläche des Visierschilds (9) oder des Brillenglases (8) ist und die Stärke der Grundmaterialfolie (100) 0,2 mm oder mehr und 0,6 mm oder weniger beträgt.

3. Befestigungsfolie (10) nach Anspruch 1 oder 2, wobei die Antibeschlagsschicht (105) eine hydrophile Antibeschlagsfolie ist, die chemisch mit der einen Oberfläche der Grundmaterialfolie (100) verbunden ist.

4. Helm (1), umfassend:
einen Visierschild (9);
eine Befestigungsfolie (10) zur Anbringung auf einer konkaven gekrümmten Innenwandfläche des Visierschilds (9); und
Befestigungsmittel (20, 120) zum Befestigen der Befestigungsfolie (10) auf der konkaven gekrümmten Innenwandfläche des Visierschilds (9);
wobei die Befestigungsfolie (10) eine Befestigungsfolie (10) nach einem der Ansprüche 1 bis 3 ist; und
wobei das Befestigungsmittel (20, 120) zumindest an beiden Enden in einer Längsrichtung der Befestigungsfolie (10) vorgesehen ist und die Befestigungsfolie (10) am Visierschild (9) an den beiden Enden befestigt.

5. Helm nach Anspruch 4, wobei das Befestigungsmittel eine elastische Haftschicht (120) ist, die zwischen dem Visierschild (9) und der Befestigungsfolie (10) angeordnet ist und eine konvexe gekrümmte Oberfläche auf einer Oberfläche aufweist, die zumindest einem des Visierschilds (9) und der Befestigungsfolie (10) zugekehrt ist.

6. Helm nach Anspruch 4, wobei der Visierschild (9) aus Polycarbonat ausgebildet ist und das Befestigungsmittel (20, 120) beide Enden der Befestigungsfolie (10) in einem durchgebogenen Zustand in Sandwichbauweise einlegt und befestigt.

7. Helm nach Anspruch 6, wobei das Befestigungsmittel zumindest eine elastische Haftschicht (120) ist, die fortlaufend entlang eines Außenkantenabschnitts der Befestigungsfolie (10) vorgesehen ist.

8. Helm nach Anspruch 7, wobei das Befestigungsmittel eine elastische Haftschicht (120) ist, die die hoch erhoben ist oder eine große Breite an beiden Längsendenabschnitten der Befestigungsfolie (10) statt an einem Längsmittelabschnitt der Befestigungsfolie (10) aufweist.

9. Brille, umfassend:
ein Brillenglas (8);
eine Befestigungsfolie (10) zur Anbringung auf einer konkaven gekrümmten Innenwandfläche des Brillenglases (8); und
Befestigungsmittel (20, 120) zum Befestigen der Befestigungsfolie (10) auf der konkaven gekrümmten Innenwandfläche des Brillenglases (8);
wobei die Befestigungsfolie (10) eine Befestigungsfolie (10) nach einem der Ansprüche 1 bis 3 ist; und
wobei das Befestigungsmittel (20, 120) zumindest an beiden Enden in einer Längsrichtung der Befestigungsfolie (10) vorgesehen ist und die Befestigungsfolie (10) am Brillenglas (8) an den beiden Enden befestigt.

10. Verfahren zum Anbringen einer Antibeschlagsfolie (105), die folgenden Schritte umfassend:
Ausbilden einer Antibeschlagsschicht (105) auf zumindest einer Oberfläche einer Polycarbonatgrundmaterialfolie (100), die eine eine Stärke von 0,2 mm oder mehr und 1,0 mm oder weniger aufweist; und
Befestigen der Grundmaterialfolie (100) auf einer konkaven gekrümmten Innenwandfläche des Visierschilds (9) oder des Brillenglases (8) in einem Zustand, in dem die Grundmaterialfolie (100) durchgebogen ist, sodass die Oberfläche, die mit der Antibeschlagsschicht (105) ausgebildet ist, eine konkave gekrümmte Oberfläche ist;
wobei eine Gesamtstärke der Grundmaterialfolie (100) und der Antibeschlagsschicht (105) 5,0 mm oder weniger beträgt.

## Revendications

1. Feuille de montage (10) destinée à être montée sur une surface de paroi interne incurvée concave d'une visière (9) ou d'un verre de lunettes (8) dans un état dans lequel la feuille de montage (10) est déviée le long de la surface incurvée, comprenant :
une feuille de matériau de base (100) qui a une courbure inférieure à celle de la surface de paroi interne incurvée concave de la visière (9) ou du verre de lunettes (8) lorsqu'elle n'est pas montée sur la surface de paroi interne incurvée concave de la visière (9) ou du verre de lunettes (8) ; et
**caractérisée en ce que** :
la feuille de matériau de base (100) est formée à partir d'un polycarbonate qui a une épaisseur de 0,2 mm ou plus et 1,0 mm ou moins ; et
la feuille de montage (10) comprend en outre une couche anti-buée (105) prévue au moins sur l'une des deux surfaces de la feuille de matériau de base (100), qui ne fait pas face à la surface de paroi interne incurvée concave de la visière (9) ou du verre de lunettes (8) lorsqu'elle est montée sur la surface de paroi interne incurvée concave de la visière (9) ou du verre de lunettes (8),
dans laquelle une épaisseur totale de la feuille de matériau de base (100) et de la couche anti-buée (105) est de 5,0 mm ou moins.

2. Feuille de montage (10) selon la revendication 1, dans laquelle la surface de paroi interne incurvée concave de la visière (9) ou du verre de lunettes (8) est une surface de paroi interne incurvée concave tridimensionnelle lisse de la visière (9) ou du verre de lunettes (8), et l'épaisseur de la feuille de matériau de base (100) est de 0,2 mm ou plus et 0,6 mm ou moins.

3. Feuille de montage (10) selon la revendication 1 ou 2, dans laquelle la couche anti-buée (105) est un film anti-buée hydrophile chimiquement relié à ladite une surface de la feuille de matériau de base (100).

4. Casque (1) comprenant :
une visière (9) ;
une feuille de montage (10) destinée à être montée sur une surface de paroi interne incurvée concave de la visière (9) ; et
un moyen de fixation (20, 120) pour fixer la feuille de montage (10) sur la surface de paroi interne incurvée concave de la visière (9) ;
dans lequel la feuille de montage (10) est une feuille de montage (10) selon l'une des revendications 1 à 3 ; et
dans lequel le moyen de fixation (20, 120) est au moins prévu aux deux extrémités dans une direction longitudinale de la feuille de montage (10) et fixe la feuille de montage (10) sur la visière (9) au niveau des deux extrémités.

5. Casque selon la revendication 4, dans lequel le moyen de fixation est une couche adhésive élastique (120) disposée entre la visière (9) et la feuille de montage (10) et ayant une surface incurvée convexe sur une surface faisant face au moins l'une parmi la visière (9) et la feuille de montage (10).

6. Casque selon la revendication 4, dans lequel la visière (9) est formée à partir de polycarbonate, et le moyen de fixation (20, 120) prend en sandwich et fixe les deux extrémités de la feuille de montage (10) dans un état dévié.

7. Casque selon la revendication 6, dans lequel le moyen de fixation est au moins une couche adhésive élastique (120) prévue de manière continue, le long d'une partie de bord externe de la feuille de montage (10).

8. Casque selon la revendication 7, dans lequel le moyen de fixation est une couche adhésive élastique (120) qui est très haute ou a une grande largeur aux deux parties d'extrémité longitudinales de la feuille de montage (10) plutôt qu'au niveau d'une partie centrale longitudinale de la feuille de montage (10).

9. Lunettes comprenant :
un verre de lunettes (8) ;
une feuille de montage (10) destinée à être montée sur une surface de paroi interne incurvée concave du verre de lunettes (8) ; et
un moyen de fixation (20, 120) pour fixer la feuille de montage (10) sur la surface de paroi interne incurvée concave du verre de lunettes (8) ; et
dans lesquelles la feuilles de montage (10) est une feuille de montage (10) selon l'une des revendications 1 à 3 ; et
dans lesquelles le moyen de fixation (20, 120) est au moins prévu au niveau des deux extrémités dans une direction longitudinale de la feuille de montage (10) et fixe la feuille de montage (10) sur le verre de lunettes (8) au niveau des deux extrémités.

10. Procédé pour monter une feuille anti-buée (105) comprenant les étapes consistant à :
former une couche anti-buée (105) sur au moins une surface d'une feuille de matériau de base en polycarbonate (100) ayant une épaisseur de 0,2 mm ou plus et 1,0 mm ou moins ; et
fixer la feuille de matériau de base (100) sur une surface de paroi interne incurvée concave d'une visière (9) ou du verre de lunettes (8) dans un état dans lequel la feuille de matériau de base (100) est déviée de sorte que la surface formée avec la couche anti-buée (105) est une surface incurvée concave ;
dans lequel une épaisseur totale de la feuille de matériau de base (100) et de la couche anti-buée (105) est de 5,0 mm ou moins.
